# EUROPÄISCHE PATENTSCHRIFT

(11) **EP 0 538 729 B1**
(45) Veröffentlichungstag und Bekanntmachung des Hinweises auf die Patenterteilung: **10.04.1996**
(21) Anmeldenummer: 92117579.0
(22) Anmeldetag: 15.10.1992
(51) Int. Cl.: C07C 35/08, C07C 29/04

(54) **Verfahren zur Herstellung von Cycloalkanolen**
Process for the preparation of cycloalkanols
Procédé de préparation de cycloalcanols

(30) Priorität: 25.10.1991 DE 4135238
(43) Veröffentlichungstag der Anmeldung: 28.04.1993
(73) Patentinhaber: BASF Aktiengesellschaft, D-67063 Ludwigshafen (DE)
(72) Erfinder: Pinkos, Rolf, Dr., W-6702 Bad Duerkheim (DE); Fischer, Rolf, Dr., W-6900 Heidelberg (DE)

(56) Entgegenhaltungen:
- GB-A- 2 151 224
- CHEMICAL ABSTRACTS, vol. 106, no. 20, 1987, Columbus, Ohio, US; abstract no. 158284, Seite 108 ;Spalte 1 ; & JP-A-61 234 945

## Beschreibung

Die Erfindung betrifft ein Verfahren zur Herstellung von Cycloalkanolen durch Umsetzen von Cycloolefinen mit Wasser in Gegenwart von sauren Katalysatoren.

In der DE-PS 34 41 072 wird ein Verfahren zur Herstellung von cyclischen Alkohlen beschrieben, bei dem man Cycloolefine mit Wasser in flüssiger Phase bei einer Temperatur von 50 bis 250°C in Gegenwart von Zeolithen mit einem Verhältnis von sauren Stellen an der äußeren Oberfläche zur Gesamtzahl von sauren Stellen ≧ 0,07 und einer primären Teilchengröße < 0,5 µm umsetzt. Die Umsetzung wird mit einem suspendierten Zeolith-Katalysator durchgeführt. Das Verfahren hat den Nachteil, daß der pulverförmige Katalysator nach der Umsetzung abgetrennt werden muß, was aufwendig und zudem eine Schädigung des Katalysators nicht auszuschließen ist.

Ferner ist aus der EP-A-162 475 bekannt, daß man Cycloolefine vorteilhaft hydratisiert, wenn man Zeolithe, die Titan, Zirkon, Hafnium, Chrom, Molybdän, Wolfram oder Torium als Bausteine enthalten und wäßrige Säuren verwendet. Dieses Verfahren hat den Nachteil, daß die wäßrigen Säuren wiedergewonnen werden müssen.

Aus Chem.Abs.106,no.158284 (1987) ist ein Verfahren zur Herstellung von Cycloalkanolen durch Umsetzung eines Gemisches aus Cycloolefinen und Wasser bekannt, wobei das Reaktionsgemisch mit dem Katalysator eine Suspension bildet. In der GB-A 2,151,224 ist ebenfalls ein Verfahren zur katalytischen Addition von Wasser an cyclische Olefine in der Flüssigphase beschrieben. In beiden Verfahren muß das Cycloalkanol in einem zusätzlichen Schritt vom eingesetzten Katalysator getrennt werden.

Es war deshalb die technische Aufgabe gestellt, ein Verfahren zur Herstellung von Cycloalkanolen durch Hydratisieren von Cycloolefinen zur Verfügung zu stellen, bei dem das Cycloalkanol enthaltende Reaktionsgemisch frei von Katalysator anfällt, der Katalysator nicht geschädigt wird und leicht regenerierbar ist, keine zusätzlichen flüssigen Säuren, die zurückgewonnen werden müßen, verwendet werden und zudem möglichst Nebenreaktionen wie Polymerbildung oder andere Nebenreaktionen vermieden werden.

Diese Aufgabe wird gelöst in einem Verfahren zur Herstellung von Cycloalkanolen durch Umsetzen von Cycloolefinen mit Wasser in Gegenwart von festen sauren Katalysatoren bei einer Temperatur von 50 bis 280°C und unter erhöhtem Druck mit der Maßgabe, daß die Temperatur- und Druckbedingungen zur Ausbildung flüssiger Phasen aus Wasser und Cycloolefin ausreichen, dadurch gekennzeichnet, daß man ein Gemisch aus Wasser und Cycloolefin durch eine fest angeordnete Schicht von festen sauren Katalysatoren leitet, mit der Maßgabe, daß sich in der Schicht aus festen sauren Katalysatoren keine sichtbare flüssige Phase ausbildet.

Das neue Verfahren hat den Vorteil, daß das Reaktionsgemisch bereits vom Katalysator getrennt anfällt. Weiter hat das neue Verfahren den Vorteil, daß der Katalysator leicht regenierbar ist und keine zusätzlichen flüssigen Säuren, die zurückgewonnen werden müßen, verwendet werden. Ferner hat das neue Verfahren den Vorteil, daß Nebenreaktionen minimiert werden.

Bevorzugte Cycloolefine haben 5 bis 8 Kohlenstoffatome im Ring und eine olefinische Doppelbindung. Geeignete Cycloolefine sind beispielsweise Cyclopenten, Cyclohexen, Cycloocten. Besondere Bedeutung hat Cyclohexen als Ausgangsstoff erlangt. Es ist auch möglich, Gemische aus Cyclohexen, Cyclohexan und Benzol, wie sie bei der partiellen Hydrierung Von Benzol anfallen, zu verwenden und durch Hydratisieren Cyclohexen unter Bildung von Cyclohexanol abzutrennen.

Die Cycloolefine werden mit Wasser umgesetzt. Vorteilhaft verwendet man je Mol Cycloolefin 100 bis 0,04 Mol Wasser. Besonders bewährt hat es sich, wenn man je Mol Cycloolefin 50 bis 0,1, insbesondere 10 bis 0,5 Mol Wasser anwendet.

Die Umsetzung wird bei einer Temperatur von 50 bis 280°C, vorteilhaft von 70 bis 230°C, insbesondere von 90 bis 180°C durchgeführt. Ferner wendet man bei der Umsetzung erhöhten Druck an. In der Regel beträgt der Druck 1 bis 250 bar und vorteilhaft 2 bis 150 bar, insbesondere 4 bis 80 bar. Die Druck- und Temperaturbedingungen werden so aufeinander abgestimmt, daß sie zur Ausbildung flüssiger Phasen aus Wasser und Cycloolefin ausreichen.

Die Umsetzung wird in Gegenwart von festen sauren Katalysatoren durchgeführt. Geeignete feste saure Katalysatoren sind beispielsweise stark saure Ionenaustauscher wie Sulfonsäuregruppen enthaltendes vernetztes Polystyrol, eine andere Gruppe von festen sauren Katalysatoren sind saure, praktisch wasserunlösliche Oxide, wie ZrO₂, SnO₂, TiO₂, die gegebenenfalls mit zusätzlichen sauren Gruppen wie SO₄²⁻ dotiert sein können. Bevorzugte Katalysatoren sind Zeolithe. Bevorzugt sind hiervon Zeolithe in der H-Form, beispielsweise aus der Mordenit-Gruppe oder engporige Zeolithe wie die X-, Y- oder L-Zeolithe, z.B. Mordenit, Erionit, Chabasit oder Faujasit. Ferner sind geeignet ultrastabile Zeolithe aus der Faujasit-Gruppe, die dealuminiert sind.

Besonders vorteilhaft sind Zeolithe mit Pentasilstruktur wie ZSM-5, ZSM-11 und ZBM-10. Diese haben als Grundbaustein einen aus SiO2-Tetraedern aufgebauten Fünfring gemeinsam, weisen ein hohes Verhältnis Von Siliciumdioxid zu Aluminiumoxid auf und haben Porengrößen, die zwischen denen der Zeolithe vom Typ A und denen vom Typ X oder Y liegen. Es hat sich bewährt, wenn in den Alumozeolithen das Verhältnis von Siliciumdioxid zu Aluminiumoxid < 100 beträgt, vorteilhaft von 30 bis 50, insbesondere von 70 bis 90.

Die Katalysatoren werden im allgemeinen als Stränge, Kugeln oder Pulver angewandt. Da eine dichte Packung und große Oberfläche bevorzugt sind, eignet sich pulverförmiger Katalysator besonders gut, insbesondere mit einer Teilchengröße von 0,1 bis 1 µm.

Die festen sauren Katalysatoren werden in einer fest angeordneten Schicht angewandt, z.B. in einer zylinderischen Schicht. Der feste saure Katalysator wird z.B. als Pulver entweder trocken in einer rohrförmige Reaktionszone eingebracht oder im Wasser oder im Cycloolefin suspendiert eingeschlämmt. Ein Austrag des Katalysators wird beispielsweise durch eine Fritte oder ein geeignetes Filter verhindert.

Durch die fest angeordnete Schicht von festen sauren Katalysatoren wird zweckmäßig von oben Cycloolefin und Wasser flüssig aufgegeben. Die beiden Komponenten bedürfen keiner innigen Durchmischung. Wasser und Cycloolefine werden nun unter Druck durch die fest angeordnete Katalysatorschicht geleitet, mit der Maßgabe, daß sich in der Katalysatorschicht keine sichtbare flüssige Phase ausbildet. Dies wird dadurch erreicht, daß man gerade so viel Wasser und Cycloolefin zugibt, daß diese an der Katalysatorschicht absorbiert werden. Am unteren Ende der Katalysatorschicht erhält man eine wäßrige Phase, die geringe Menge, z.B. bis zu 2 Gew.-% Cycloalkanol enthält und eine organische Phase, die im wesentlichen aus nichtumgesetztem Cycloolefin und Cycloalkanol besteht.

In der Regel führt man 10 bis 0,01 Gewichtsteile eines Gemisches aus Cycloolefin und Wasser je Gewichtsteil festen sauren Katalysator und Stunde zu. Besonders bewährt hat es sich, 5 bis 0,1 Gewichtsteile eines Gemisches aus Wasser und Cycloolefin je Gewichtsteil sauren festen Katalysator und Stunde anzuwenden.

Bei einer kontinuierlichen Arbeitsweise wird wäßrige Phase nach Ergänzung der Wassermenge wiederum auf die Katalysatorschicht aufgegeben, während aus der organischen Phase durch Destillation Cycloolefin abgetrennt und nach Ergänzung ebenfalls wieder auf die Katalysatorschicht aufgegeben wird und so Cycloalkanol ausgeschleust wird.

Sofern der Katalysator z.B. der Zeolith im Verlauf der Reaktion desaktiviert wird, läßt er sich auf einfache Weise regenerieren. Hierbei wird im allgemeinen die fest angeordnete Schicht aus festen saurem Katalysator zunächst mit Wasser gespült und dann bei einer Temperatur von 50 bis 120°C, insbesondere 65 bis 80°C mit einer wäßrigen Wasserstoffperoxid-Lösung beaufschlagt. Geeignete Wasserstoffperoxid-Lösungen haben einen Gehalt an H₂O₂ von 0,1 bis 30 Gew.-%, insbesondere von 3 bis 15 Gew.-%. Im Verlauf der Regenerierung verfolgt man durch Messen des pH-Wertes, der zu Beginn der Regnerierung z.B. 1 bis 4 beträgt und im Verlauf der Regenierung auf pH 7 ansteigt. Anstelle von Wasserstoffperoxid können für die Regenerierung auch andere oxidierende Mittel, wie Ozon, molekularen Sauerstoff, Luft oder Peroxoverbindungen, z.B. Peroxosulfate verwendet werden.

Das Verfahren nach der Erfindung sei an folgenden Beispielen veranschaulicht.

### Beispiel 1

Ein 50 ml Rohrreaktor wurde mit 15,9 g eines ZSM-11- Zeolithen als Aufschlämmung in Wasser teilweise gefüllt. Von oben wurden 0,5 g Wasser und 0,9 g Cyclohexen/Minute bei 127°C (Temperatur im Festbett) aufgegeben. Dies entsprach 5 Gewichtsteile Wasser und Cyclohexen je Gewichtsteil Zeolith und Stunde. Am Kopf des Reaktors wurde ein Druck von 15 bar erreicht.

Nach 4 h Reaktionszeit betrug der Gehalt der organischen Phase im Austrag 9 Gew.-% Cyclohexanol, nach 8 h 11 %. Danach sank der Umsatz kontinuierlich ab und erreichte nach 34 h 5 %. In der Wasserphase fanden sich zwischen 1 und 2 Gew.-% Cyclohexanol. Zur Regenerierung des Zeolithen wurden nach 12-stündigen Spülen des Reaktors mit Wasser bei 70 - 80°C 1,5 ml einer 5%igen wässrigen H₂O₂-Lösung/Minute zugefahren. Der wässrige Austrag wies eine dunkle Färbung auf und der pH lag zwischen 2 und 3. Nach 13 h war der Austrag wieder farblos und der pH lag zwischen 6 - 7. Nach Aufheizen auf 127°C und 5 h Wasserspülung konnten unter den oben erläuterten Bedingungen die Ausgangsumsätze wieder erreicht werden.

### Beispiel 2

Ein 150 ml Rohrreaktor wurde mit 76 g eines ZSM-5-Zeolithen als Aufschlämmung in Wasser teilweise gefüllt und der Zeolith 12 h mit 1 ml Wasser/Minute gepült. Anschließend wurden Wasser und Cyclohexen in einem Molverhältnis von 1 : 2 bei 129°C in einer Menge von 1 Gewichtsteil Cyclohexen und Wasser je Gewichtsteil Katalystor und Stunde über das Festbett gefahren. Der maximale Reaktionsdruck betrug 35 bar. Es wurden bis 10 Gew.-% Cyclohexanol in der organischen Phase gebildet. In der Wasserphase befanden sich ca. 2 % Cyclohexanol.

### Beispiel 3

Analog Beispiel 2 wurde ein Molverhältnis Wasser : Cyclohexen von 1,4 : 1 verwendet. Bei 0,8 Gewichtsteilen Cyclohexen und Wasser je Gewichtsteil Katalysator und Stunde wurden 10-11 Gew.-%, bei 0,4 Gew.-Teilen Cyclohexen und Wasser je Gew.-Teil Katalysator und Stunde wurden 14 Gew.-% Cyclohexanol in der organischen Phase gefunden. In der Wasserphase befanden sich ca. 2 % Cyclohexanol.

### Beispiel 4

Analog Beispiel 3 wurde ein Molverhältnis Wasser : Cyclohexen von 1 : 1 verwendet. Bei 0,7 Gew.-Teilen Cyclohexen und Wasser je Gew.-Teil Katalysator und Stunde wurden bei 160°C 13 Gew.-% Cyclohexanol in der organischen Phase gefunden. Dabei wurde die Wasserphase zurückgeführt und das verbrauchte Wasser ergänzt. Die organische Phase wurde destillativ in Cyclohexen und Cyclohexanol aufgetrennt. Das Cyclohexen wurde ebenfalls zurückgeführt. Destillation des Cyclohexanols führte zu einer Reinheit von 99,9 % (GC). Als Rückstand blieben geringe Mengen Cyclohexylcyclohexen.

Nach 180 h Reaktionszeit zeigte der Katalysator noch keine Desaktivierung. Durch die Rückführung von Cyclohexen reicherte sich etwas Methylcyclopenten an, welches einerseits schon im eingesetzten Cyclohexen nachweisbar war, andererseits während der Reaktion gebildet wurde (als Summe jedoch < 0,5 %).

### Beispiel 5

In einen 25 ml Glasautoklaven wurden 1,5 g eines ZSM-11-Zeolithen, 0,01 Mol Cyclohexen und 0,04 Mol Wasser gefüllt (Gewichtsverhältnis Zeolith : Summe Cyclohexen/Wasser = 1 : 1). Das Reaktiongefäß wurde mit Argon gespült und dann 1 h auf 120°C erhitzt. Dabei stellte sich ein Druck von 3 - 4 bar ein. Der Reaktorinhalt zeigte weder vor, während, noch nach der Reaktion eine flüssige Phase. Nach der Reaktion wurde der Reaktorinhalt mit Aceton versetzt, um die Reaktionskomponenten vom Katalysator zu trennen. GC-analytisch konnten 17 Mol-% Cyclohexanol bezogen auf eingesetztes Cyclohexen nachgewiesen werden. Nebenprodukte wurden nicht gefunden.

### Vergleichsbeispiel

Analog Beispiel 5 wurden in einen 25 ml Glasautoklaven 1 g eines ZSM-11-Zeolithen, 0,01 Mol Cyclohexen und 0,1 Mol Wasser gefüllt (Gewichtsverhältnis Zeolith : Summe Cyclohexen/Wasser = 1 : 2,6). Das Reaktiongefäß wurde mit Argon gespült und dann 2 h auf 120°C ohne Rühren erhitzt. Dabei stellte sich ein Druck von 4 - 5 bar ein. Der Reaktorinhalt zeigte vor, während und nach der Reaktion zwei flüssige Phasen, wobei sich der Katalysator vorwiegend in der unteren (wässrigen) Phase befand. Nach der Reaktion wurden die flüssigen Phasen mit Aceton homogenisiert. GC- analytisch konnte nur 1 Mol-% Cyclohexanol nachgewiesen werden.

## Patentansprüche

1. Verfahren zur Herstellung von Cycloalkanolen durch Umsetzen von Cycloolefinen mit Wasser in Gegenwart von festen sauren Katalysatoren bei einer Temperatur von 50 bis 280°C, mit der Maßgabe, daß die Druck- und Temperaturbedingungen zur Ausbildung einer flüssigen Phase aus Wasser und Cycloolefinen ausreichen, dadurch gekennzeichnet, daß man bei einem Druck von 1 bis 250 bar ein Gemisch aus Wasser und Cycloolefin durch eine fest angeordnete Schicht von festen sauren Katalysatoren leitet, mit der Maßgabe, daß sich in der Schicht aus festen sauren Katalysatoren keine sichtbare flüssige Phase ausbildet.

2. Verfahren nach Anspruch 1, dadurch gekennzeichnet, daß man 50 bis 0,1 Mol Wasser je Mol Cycloolefin anwendet.

3. Verfahren nach den Ansprüchen 1 und 2, dadurch gekennzeichnet, daß man 10 bis 0,01 Gewichtsteile eines Gemisches aus Wasser und Cycloolefin je Gewichtsteil festen sauren Katalysator und Stunde anwendet.

4. Verfahren nach den Ansprüchen 1 bis 3, dadurch gekennzeichnet, daß man eine Temperatur von 70 bis 230°C einhält.

5. Verfahren nach den Ansprüchen 1 bis 4, dadurch gekennzeichnet, daß man einen Druck von 1 bis 250 bar einhält.

6. Verfahren nach den Ansprüchen 1 bis 5, dadurch gekennzeichnet, daß man Zeolithe in der H-Form als feste saure Katalysatoren verwendet.

7. Verfahren nach den Ansprüchen 1 bis 6, dadurch gekennzeichnet, daß man die fest angeordnete Schicht aus festen sauren Katalysatoren nach Desaktivierung mit einer wäßrigen Lösung von Wasserstoffperoxid regeneriert.

8. Verfahren nach den Ansprüchen 1 bis 7, dadurch gekennzeichnet, daß man den Verlauf der Regenerierung durch pH-Messung des Austrags verfolgt.

9. Verfahren nach den Ansprüchen 1 bis 7, dadurch gekennzeichnet, daß man Cyclohexen als Cycloolefin verwendet.

## Claims

1. A process for preparing cycloalkanols by reacting cycloolefins with water in the presence of solid acidic catalysts at a temperature from 50 to 280°C, with the proviso that the pressure and temperature conditions are sufficient to form a liquid phase of water and cycloolefins, characterized in that a mixture of water and cycloolefin is passed through a fixed bed of solid acidic catalysts at a pressure from 1 to 250 bar, with the proviso that no visible liquid phase forms in the bed of solid acidic catalysts.

2. A process as claimed in claim 1, characterized in that from 50 to 0.1 mol of water are used per mole of cycloolefin.

3. A process as claimed in claims 1 and 2, characterized in that from 10 to 0.01 parts by weight of a mixture of water and cycloolefin are used per part by weight of solid acidic catalyst per hour.

4. A process as claimed in any of claims 1 to 3, characterized in that a temperature from 70 to 230°C is maintained.

5. A process as claimed in any of claims 1 to 4, characterized in that a pressure from 1 to 250 bar is maintained.

6. A process as claimed in any of claims 1 to 5, characterized in that the solid acidic catalysts used are zeolites in the H-form.

7. A process as claimed in any of claims 1 to 6, characterized in that the fixed bed of solid acidic catalysts is regenerated with an aqueous solution of hydrogen peroxide after deactivation.

8. A process as claimed in any of claims 1 to 7, characterized in that the course of the regeneration is monitored by pH measurement of the effluent.

9. A process as claimed in any of claims 1 to 7, characterized in that the cycloolefin used is cyclohexene.

## Revendications

1. Procédé de préparation de cycloalcanols par réaction de cyclooléfines avec de l'eau en présence de catalyseurs acides solides, à une température de 50 à 280°C, étant spécifié que les conditions de pression et de température sont suffisantes pour la formation d'une phase liquide d'eau et de cyclooléfines, caractérisé en ce que l'on fait passer, sous une pression de 1 à 250 bar, un mélange d'eau et de cyclooléfine à travers une couche en lit fixe de catalyseur acide solide, étant spécifié qu'il ne se forme pas de phase liquide visible dans la couche de catalyseur acide solide.

2. Procédé selon la revendication 1, caractérisé en ce que l'on utilise de 50 à 0,1 moles d'eau par mole de cyclooléfine.

3. Procédé selon la revendication 1 ou 2, caractérisé en ce que l'on utilise de 10 à 0,01 parties en poids d'un mélange d'eau et de cyclooléfine par partie en poids de catalyseur acide solide et par heure.

4. Procédé selon l'une quelconque des revendications 1 à 3, caractérisé en ce que l'on maintient une température de 70 à 230°C.

5. Procédé selon l'une quelconque des revendications 1 à 4, caractérisé en ce que l'on maintient une pression de 1 à 250 bar.

6. Procédé selon l'une quelconque des revendications 1 à 5, caractérisé en ce que l'on utilise, comme catalyseurs acides solides, des zéolithes sous la forme H.

7. Procédé selon l'une quelconque des revendications 1 à 6, caractérisé en ce qu'après la désactivation de la couche en lit fixe de catalyseur acide solide, on la régénère avec une solution aqueuse de peroxyde d'hydrogène.

8. Procédé selon l'une quelconque des revendications 1 à 7, caractérisé en ce que l'on suit le déroulement de la régénération par mesure du pH de l'effluent.

9. Procédé selon l'une quelconque des revendications 1 à 7, caractérisé en ce que l'on utilise du cyclohexène comme cyclooléfine.
